(19) Europäisches Patentamt
European Patent Office
Office européen des brevets

(11) Numéro de publication: **0 252 060 A1**

(12) **DEMANDE DE BREVET EUROPEEN**

(21) Numéro de dépôt: **87870091.3**

(22) Date de dépôt: **29.06.87**

(51) Int. Cl.4: **C 12 N 15/00**
C 12 P 21/02, A 61 K 39/17

(30) Priorité: **30.06.86 US 880371**

(43) Date de publication de la demande: **07.01.88 Bulletin 88/01**

(84) Etats contractants désignés: **AT BE CH DE ES FR GB GR IT LI LU NL SE**

(71) Demandeur: **Smith Kline - RIT Société anonyme dite:**
**rue du Tilleul, 13**
**B-1320 Genval (Rixensart) (BE)**

(72) Inventeur: **Espion, Danièle**
**Rue Colonel Chaltin, 2a, bte 3**
**B-1180 Bruxelles (BE)**

**Burny, Arsène**
**Chaussée de Namur, 65**
**B-5800 Gembloux (BE)**

**Debouck, Christine**
**667 Pugh Road**
**Strafford, PA. 19087 (US)**

(74) Mandataire: **Tasset, Gérard**
**SMITHKLINE - RIT rue de l'Institut, 89**
**B-1330 Rixensart (BE)**

(54) **Séquence d'ADN, molécules d'ADN recombinantes et procédé pour la production de la protéine de fusion du virus de la maladie de Newcastle ou de polypeptides équivalents, produits obtenus et préparation les contenant.**

(57) La séquence nucléotidique codant pour la protéine de fusion du virus de la maladie de Newcastle ou pour un polypeptide équivalent est déterminée, le produit de fusion est exprimé par exemple dans E. coli ou dans une levure; la séquence est utilisable dans un but vaccinal ou de diagnostic.

EP 0 252 060 A1

## Description

Séquence d'ADN, molécules d'ADN recombinantes et procédé pour la production de la protéine de fusion du virus de la maladie de Newcastle ou de polypeptides équivalents, produits obtenus et préparation les contenant

Domaine de l'invention

La présente invention concerne une séquence d'ADN, des molécules d'ADN recombinantes et un procédé pour la production de la protéine de fusion du virus de la maladie de Newcastle ou de polypeptides équivalents, les produits obtenus et les préparations les contenant, plus particulièrement pour des buts de diagnostic ou de vaccination.

Arrière-plan technologique

La maladie de Newcastle, parfois appelée 'peste aviaire' est l'une des maladies les plus importantes parmi celles qui affectent la volaille. L'agent responsable est un paramyxovirus : le virus de la maladie de Newcastle (NDV). L'infection virale provoque l'apparition de symptômes respiratoires parfois accompagnés de sévères complications nerveuses conduisant à une paralysie et à la mort, causant de graves difficultés économiques dans l'industrie d'élevage de la volaille.

La plupart des vaccins contre cette maladie sont préparés avec des virus peu virulents ou atténués; néanmoins, les vaccins ainsi préparés ne sont pas entièrement sans danger car ils peuvent encore induire quelques maladies respiratoires qui se compliquent parfois d'infections secondaires. C'est pourquoi leur usage ne constitue pas une forme idéale de contrôle de l'infection. C'est donc un objet de l'invention de préparer un vaccin efficace et sans danger par la production, par techniques de génie génétique, de quantités substantielles de protéines virales responsables de l'induction d'anticorps neutralisants lors de l'infection.

Des candidats pour un tel vaccin étaient les deux glycoprotéines présentes dans la membrane du virus, c'est-à-dire NH et F (Ray R. et al. : J. Inf. Dis. 152; 1219-1230; 1985). Les deux protéines forment des projections de type spicule hérissant la face externe de la membrane du virus et, ensemble, elles interviennent dans les interactions virales au niveau de la membrane de la cellule : NH est responsable de l'adsorption du virus tandis que F intervient dans la fusion des membranes virale et cellulaire. La protéine de fusion, F, est essentielle pour l'infectivité du virus (Nagai Y. et al. : Virology 72; 494-508; 1976) et sa neutralisation est nécessaire pour une prévention immunologique effective d'une infection par paramyxovirus (Merz, D.C. et al. : J. Exp. Med. 151; 275-288; 1980).

Il est bien établi que la protéine F est d'abord synthétisée sous forme d'un précurseur inactif ($F_0$ ) et est ensuite clivée de manière spécifique par une enzyme protéolytique de l'hôte de manière à fournir deux polypeptides liés par ponts disulfure ($F_1$ et $F_2$ ) (Nagai Y. et al. loc. cit.; Nagai Y. et al. Virology 77; 125-134; 1977). Ce clivage intracellulaire (Morrisson T. et al. J. Virol. 53; 851-857; 1985) génère un nouveau groupement amino sur le polypeptide $F_1$ et s'est avéré essentiel pour l'activité biologique de F (Nagai Y. et al. loc. cit.). La séquence d'acides aminés de la région N-terminale de $F_1$ est très hydrophobe et fortement conservée parmi plusieurs paramyxovirus (Choppin P.A. et al. J. Inf. Dis. 143; 352-363; 1981). Ces constatations ont suggéré que cette nouvelle région amino-terminale pourrait interférer avec la membrane cellulaire cible dans la réaction de fusion membranaire. Cette possibilité a été confirmée par des études au cours desquelles des oligopeptides synthétiques, dont la séquence mime le groupement amino-terminal de $F_1$ , inhibaient spécifiquement la fusion de la membrane par plusieurs paramyxovirus (Richardson C.D. et al. Virology 131; 518-532; 1983 et Choppin P.W. et al. loc. cit.).

NH et F induisent tous deux des anticorps neutralisants (Lê Long et al. J. Virol. 57; 1198-1202; 1986). Cependant, F. paraît être un élément essentiel pour un vaccin (Merz D.C. et al. loc. cit. et Choppin P.W. et al. loc. cit.). En effet, contrairement aux anticorps anti-NH, les anticorps anti-F empêchent complètement la propagation de l'infection, par inhibition des deux types de fusion : la fusion de l'extérieur causée par les virus infectieux libérés de cellules infectées et la fusion de l'intérieur qui conduit à une propagation de l'infection de cellule à cellule par fusion de la membrane (Merz D.C. et al. loc. cit. et Choppin P.W. et al. loc. cit.). En conséquence, il a été considéré que, pour être efficace, un vaccin doit induire des anticorps contre la glycoprotéine F et que la glycoprotéine F pure serait l'immunogène idéal pour un tel vaccin (Choppin M.W. et al. loc. cit.).

On a détecté des protéines de fusion non seulement dans le virus de la maladie de Newcastle (NDV) mais aussi dans d'autres paramyxovirus (par exemple SV5 et le virus de Sendai) et dans les virus syncytiaux respiratoires humains (HRSV) et, avant cette invention, les séquences d'ADN codant pour les protéines de fusion des virus SV5, de Sendai et HRSV ont été déterminées (Paterson R.G. et al. Proc. Natl. Acad. Sci. USA 81; 6706-6710; 1984; Blumberg B.M. et al. J. Gen. Virol. 66; 317-331; 1985; Elango N. et al. Nucl. Ac. Res. 13; 1559-1574; 1985 et Collins P.L. et al. Proc. Natl. Acad. Sci. USA 81; 7683-7687; 1984).

Il est également connu que les protéines de fusion de paramyxovirus dont les séquences sont connues ont en commun un caractère général plutôt hydrophobe et présentent 3 régions hydrophobes caractéristiques, à savoir la séquence signal, l'extrémité N-terminale de $F_4$ et le domaine d'ancrage.

Avant la présente invention, la protéine F de NDV avait été détectée par électrophorèse sur gel de SDS-polyacrylamide (Nagai Y. et al. loc. cit.); cependant la structure de la protéine F était inconnue et elle ne

pouvait pas être obtenue en quantité substantielle. Une banque comprenant des séquences de gènes de NDV avait été construite contenant des clones présentant au moins une partie du gène de F mais ce gène n'avait pas été exprimé (Chambers Ph. et al. J. Gen. Virol. 67; 475-486; 1986).

Résumé de l'invention

Sous un premier aspect, l'invention consiste en une molécule d'ADN recombinante qui comprend une séquence d'ADN codant pour la protéine de fusion du virus de la maladie de Newcastle ou un fragment ou dérivé de celle-ci codant pour un polypeptide qui peut induire une réponse immunitaire à la protéine de fusion du virus de la maladie de Newcastle.

Suivant d'autres aspects, l'invention est la molécule d'ADN recombinant constituant un vecteur d'expression qui possède la séquence codante pour la protéine de fusion liée opérativement à une séquence de contrôle d'expression et un microorganisme ou une cellule ainsi transformé.

Sous d'autres aspects encore, la présente invention est la protéine de fusion de NDV préparée par techniques d'ADN recombinant et un vaccin qui comprend ladite protéine et qui induit une réponse immunitaire contre le NDV chez un volatile.

Brève description des figures

La Figure 1 (a et b) représente la séquence nucléotidique monocaténaire de la protéine de fusion du virus de la maladie de Newcastle ADNc et la séquence d'acides aminés de la protéine $F_0$ qui en est déduite. La séquence signal présumée et le domaine d'ancrage membranaire sont soulignés d'un tireté. La flèche indique le site de clivage d'activation. Le peptide de connexion présumé entre $F_2$ et $F_1$ est marqué d'une double ligne. La séquence hydrophobe précédemment déterminé à l'extrémité N-terminale de $F_1$ est soulignée. Les sites potentiels de glycosylation sont encadrés. Les nucléotides et les acides aminés sont numérotés à la fin de chaque ligne.

LaFigure 2 représente la tache Northern (Northern blot) du poly(A)$^+$ -ARNm total extrait de cellules infectées par NDV et hybridé avec des ADN de plasmide marqués au $^{32}P$ des clones 1.5 (bande 1), I.50 (bande 2), II.14 (bande 3), III.31 (bande 5), III.36 (bande 6), I.5 et I.50 (bande 7). La position des 2 ARN 18S et 28S extraits de cellules BHK-21 est indiquée comme le sont également les poly(A)$^+$ -ARNm viraux totaux (18S, 22S et 35ARNm). Les résultats obtenus permettent une nette identification de quatre groupes correspondant aux gènes L, NH, F et NP (respectivement, clones I.5, I.50, II.14 et III.30); cependant, les ARNm correspondant aux gènes M et P n'ont pas été suffisamment séparés et l'identification des clones III.31 et III.36 était ambigüe.

La Figure 3 est une carte partielle de clivage par endonucléases de restriction et la stratégie de détermination de séquençage utilisée pour le clone II.4 (F de NDV). Les nucléotides sont numérotés à partir du départ du ADNc. Les flèches indiquent la direction du séquençage et la détermination de l'étendue de la séquence dans un fragment particulier de restriction.
La région codante est soulignée d'un tireté et les parties de cette région qui se réfèrent à $F_2$ et $F_1$ sont indiquées.

La Figure 4 décrit la construction d'un vecteur plasmide de levure contenant un insert ADNc de F de NDV : pYDE1.
Le fragment BamHI-FspI du clone II.14 a été cloné dans le site PvuII du vecteur navette pCD192 contenant le promoteur Cu-MT et le terminateur CYC1. Seule l'orientation correcte de l'insert est indiquée. Les sites de restriction pertinents sont marqués; //// : les séquences pBR322; ~ ~ ~ ~ ~ ; la séquence 2 µm; ----- : la séquence F.

La Figure 5 est la tache Western (Western blot) montrant l'expression de la protéine F dans une levure. La détection de protéine virale a été effectuée en utilisant un anticorps polyclonal dirigé contre NDV (dilution : 1/1.000). La bande 1 concerne le virus de la maladie de Newcastle; la bande 2, l'extrait cellulaire de BR10/pCD192 induit et la bande 3, l'extrait cellulaire de BR10/pYDE1 induit. Le polypeptide $F_1$ natif de NDV est indiqué, comme le sont aussi la protéine F exprimée et les poids moléculaires (MW). On peut voir que deux bandes d'environ 50 kd sont présentes après induction de BR10/pYDE1 (contenant le gène F) alors qu'on ne voit aucune bande lorsque c'est la levure BR10/pCD192 (sans gène hétérologue) qui est induite.

Description détaillée de l'invention

Une banque de clones a été construite dans le plasmide bien connu pBR322, par insertion de ADNc synthétisés au départ de poly(A)$^+$ -ARNm de cellules infectées par NDV. Après identification des clones viraux par essais d'hybridation, on a déterminé la séquence d'un ADNc qui contient l'entièreté de la région codante pour la glycoprotéine de fusion. De cette manière, on a trouvé que le polypeptide encodé a une longueur de 526 acides aminés et contient 4 sites potentiels de glycosylation (un sur $F_2$ et trois sur $F_1$ ). La séquence nucléotidique monocaténaire ADNc de la protéine de fusion du virus de la maladie de Newcastle est donnée dans la Figure 1 comme l'est également la séquence d'acides aminés de la protéine F native ($F_0$ ) qui en est déduite.

De la séquence d'acides aminés déduite pour la protéine de fusion on voit que, à l'extrémité N-terminale de la protéine se situe une région, la séquence signal, qui contient plusieurs résidus hydrophobes. Elle semble être responsable de la sortie de la protéine du réticulum endoplasmique et est habituellement éliminée dans la

protéine mature par suite d'un clivage à un site spécifique (voicin d'un acide aminé présentant une petite chaîne latérale non chargée) (Von Heijne, G. Eur. J. Biochem. 133; 17-21; 1983) qui, lorsqu'il est prédit suivant les règles de Von Heijne (Von Heijne G. loc. cit.) est supposé être voisin du résidu 18 (second des deux résidus sérine). Ce clivage conduit à la protéine de fusion précurseur $F_0$ , laquelle n'est, apparemment, pas active.

Comme indiqué ci-avant, $F_0$ est clivé par voie protéolytique pour donner deux apoprotéines, $F_1$ et $F_2$ , liées ensemble par des ponts disulfure. L'extrémité N-terminale de $F_1$ a été déterminée directement par dégradation d'Edman (Choppin P.W. et al. loc. cit.) : il s'agit d'une région hydrophobe qui peut être reconnue sur la séquence complète de F de la Figure 1 (a et b) ) (Phe-Ile-Gly-Ala..., c'est-à-dire les résidus 104 et suivants). en substance, cette séquence est identique à la séquence publiée, sauf pour une position (résidu 111:Ser au lieu de Gly).

Immédiatement avant cette région hydrophobe, on a identifié plusieurs résidus basiques (Arg-Arg-Gln-Arg-Arg; c'est-à-dire les résidus 99-103). Ce peptide qui relie $F_2$ à $F_1$ peut être éliminé pendant l'activation de la protéine de fusion comme cela a été déduit par ailleurs d'un glissement du point isoélectrique de $F_0$ vers une valeur acide, parallèlement au clivage (Kohama T. et al. Virology 111; 364-376; 1981)>

A l'extrémité carboxy de la séquence de la protéine F de NDV se trouve une région non chargée (résidue 480-512) qui présente le plus fort caractère hydrophobe de l'ensemble de la protéine et elle est flanquée de chaque côté de domaines fortement chargés. Ces particularités sont caractéristiques d'une région active comme séquence d'arrêt de transfert, c'est-à-dire interrompant la translocation de la chaîne (initiée par la séquence signal) à travers la membrane (Davis N.G. et al. Cell 41; 607-614;1985). Cette région devrait alors vraisemblablement rester ancrée dans la double couche membranaire. Elle est suivie d'une queue cytoplasmique, une région relativement polaire de 14 acides aminés (résidus 513-516) qui interagit avec la protéine de la membrane interne, M, avant ou pendant l'assemblage du virus (Peeples, M.E. et al. J. Virol. 51; 81-90; 1984).

De la séquence complète en acides aminés de F de la Figure 1 (a et b), on peut voir que la séquence signal, l'extrémité N-terminale de $F_1$ et le domaine d'ancrage sont des régions particulièrement hydrophobes. Dans l'ensemble, la séquence de F est relativement hydrophobe.

On a démontré que la glycosylation de la protéine de fusion de NDV est complètement inhibée en présence de tunicamycine (Morrisson T.G. et al. J. Virol. 36; 171-180; 1980; Lê Long et al. loc. cit), un antibiotique qui empêche la liaison azotée des chaînes carbohydrate latérales aux résidus d'asparagine. L'examen des séquences montre quatre sites potentiels de glycosylation (Asn-X-Ser ou Asn-X-Thr) : un sur $F_2$ (résidus 72-74), et trois sur $F_1$ (résidus 189-191, 354-356 et 435-437). On sait que l'apoprotéine $F_2$ est glycosylée (Scheid A. et al. Virology 80; 54-66; 1977) et un poids moléculaire moyen de 2.100 daltons pour la chaîne carbohydrate de $F_2$ (Hunter E. et al. J. Virol. 46; 920-936; 1982) prédirait un poids moléculaire moyen ($M_2$ ) de ± 10.500 pour cette fraction, un résultat en bon accord avec la mesure précédemment déduite de gels de polyacrylamide (Scheid A. et al. loc. cit.). Quant à $F_1$ , le poids moléculaire de cette apoprotéine serait de ± 52.000 si les 3 sites de glycosylation sont glycosylés; c'est-à-dire un poids moléculaire légèrement inférieur à celui qui est observé sur gel (55.000) (Morrisson T.G. et al. loc. cit.). Il apparaît donc que tous les sites potentiels de glycosylation de F sont utilisés.

Pour exprimer la protéine de fusion du virus de la maladie de Newcastle, différente sources de virus sont accessibles (par exemple, les souches Italien, Beaudette, Ulster et Queensland). Plusieurs systèmes hôte/vecteur sont accessibles également, p. ex. la bactérie E. coli et des plasmides d'expression dans lesquels un gène hétérologue peut être exprimé sous le contrôle d'un promoteur viral ou bactérien (Harris T.J.R. Genetic Engineering 4; R. Williamson ed. Academic Press, London; 127-185; 1983); les levures S. cerevisiae et S. pombe et les vecteurs navettes qui sont également des plasmides d'expression dans lesquels un gène hétérologue peut être exprimé sous le contrôle d'un promoteur de levure (Beggs J.D. Genetic Engineering 2, R. Williamson ed. Academic Press, London; 175-203; 1981); des systèmes cellules de mammifères et vecteur dérivés de réplicons viraus (Rigby P.W.J. Genetic Engineering 3, R. Williamson ed. Academic Press, London; 83-141; 1982; Campo M.S. DNA Cloning. A practical approach 2 D.M. Glover ed. IRL Press, Oxford, Wassington D.C.; 213-238; 1985); des cellules d'insectes et systèmes dérivés de réplicons viraux (French R. et al. Science 231, 1294-1297; 1986; Smith G.E. et al. Proc. Natl. Acad. Sci. USA 82, 8404-8408; 1985); et la construction d'un virus recombinant vivant dérivé de la famille des poxyvirus (p. ex. la vaccine) et qui peuvent être utilisés eux-mêmes comme vaccins (Mackett M. et al. DNA Cloning A practical approach 2, D.M. Glover ed. IRL press, Oxford, Washington D.C. 213-238; 1985).

Parmi les organismes cités ci-avant, il a été démontré ici que E. coli, S. cerevisiae et la vaccine expriment la protéine F de NDV. De préférence, c'est S. cerevisiae qui est utilisé.

Pour l'administration du vaccin, on prépare une suspension de la préparation de polypeptide obtenue dans une solution aqueuse isotonique qui est, de préférence, additionnée d'un adjuvant adéquat pour administration intramusculaire ou sous-cutanée, comme par exemple l'adjuvant complet de Freund ou de l'hydroxyde d'aluminium, comme il est bien connu dans la technique de formulation de vaccins, et la composition est répartie dans les flacons en verre contenant par exemple de 100 à 1.000 doses effectives de vaccin. Les flacons sont lyophilisés et, ensuite, bouchés hermétiquement.

Avant administration, le vaccin est reconstitué extemporanément par addition d'eau distillée apyrogène.

L'invention est illustrée par les exemples suivants.

EXEMPLES

Exemple 1. Séquence nucléotidique de la protéine de fusion de NDV

A. MATERIAUX ET METHODES

VIRUS. Du virus de la maladie de Newcastle, souche Italien, a été obtenu de l'International Reference Laboratory, Weybridge, Royaume Uni. Il a été multiplié dans des embryons de poulets âgés de 9 à 11 jours et utilisé comme source de virus typique. Le virus a été purifié comme décrit par Lê Long et al. (loc. cit.)

EXTRACTION DE poly(A)$^+$ -ARNm DE CELLULES INFECTEES PAR NDV. Des cellules BHK-21 ont été infectées à un facteur multiplicatif de 5 à 10 UFP (Unités formant plaque) par cellule. Après une période d'adsorption de 30 minutes, les cellules ont été incubées à 37° C dans du milieu essentiel minimal de Eagle (MEM). Deux heures trente après infection, on a ajouté de l'actinomycine D à une concentration finale de 2 µg/ml et, quatre heures plus tard, les cellules ont été lavées deux fois avec un tampon TNE (0,01M Tris pH 8.3, 0,15M NaCl, 0,001M EDTA) et lysées dans du tampon TNE-SDS (0,01M Tris pH 8,3, 0,15M NaCl, 0,001M EDTA, 0,5 % SDS) contenant 400 µg/ml de protéinase K. Après addition d'un volume de phénol:chloroforme:alcool isoamylique (25:24:1), l'ADL a été passé au mixer et le mélange a été extrait deux fois au phénol:chloroforme:alcool isoamylique (1 volume). Après précipitation à l'éthanol, les acides nucléiques ont été sédimentés par centrifu gation et dissous dans un tampon d'élution (0,1M Tris pH 7,5, 0,001M EDTA, 0,5 M SDS) et le mélange a été porté à la concentration 2M en LiCl et incubé à 4° C pendant 12 heures. Après centrifugation, le culot a été dissout dans 2 ml de tampon d'hybridation (0,1M Tris pH 7,5, 0,35M NaCl, 0,001 M EDTA, 0,5 % SDS) et passé sur 1,5 ml d'une colonne oligo(dT)-cellulose. Les ADN contenant les poly(A) ont été élués avec le tampon d'élution.

Avant usage, les poly(A) -ARNm ont été testés pour leur stimulation de synthèse protéinique dans des lysats de réticulocytes de lapin (Palham H.R.B. et al. Eur. J. Biochem. 67; 247-256; 1976) et dans des oocytes de Xenopus (Gurdon J.B. et al. Nature 233; 177-182; 1971); les protéines virales synthétisées ont été détectées par immunoprécipitation à l'aide d'anticorps anti-NDV spécifiques, comme décrit par Lê Long et al. (loc. cit.).

SYNTHESE ET CLONAGE DES ADNc. En utilisant des poly(A)$^+$ de cellules infectées comme patrons et un oligo primer $(dT)_{10-12'}$ des copies d'ADN monocaténaire ont été synthétisées en utilisant la transcriptase inverse. Après dénaturation par chauffage suivi de congélation des hybrides ARNm-ADNc, les boucles terminales 3'des ADNc monocaténaires ont été utilisées comme primer par de la polymérase I d'ADN pour la synthèse du second brin. Les boucles en épingle à cheveux des ADNc bicaténaires résultants ont alors été digérées par la nucléase S1 et des résidus oligo(dC) ont été ajoutés aux extrémités 3' des ADNc pour former des queues oligo(dC). Les ADNc ont alors été insérés dans le site PstI de l'ADN du plasmide pBR322 avec queue oligo(dG) par hybridation $(dG)_4$ -$(dC)_4$ et le mélange de ligation a été utilisé pour transformer la souche MM294 de E. coli (hsdR17, c'est-à-dire $r_K^- m_K^-$) lui conférant une résistance à la tétracycline.

DETECTION DES CLONES VIRAUX. Les clones contenant des ADNc viraux comme inserts (199 colonies parmi 1277 résistantes à la tétracycline et sensibles à l'ampicilline) ont été détectés par hybridation des colonies in situ (Grunstein M. et al. loc. cit.) en utilisant comme sonde de l'ARN viral 50S marqué au $^{32}P$ (isolé sur gradient de saccharose (15-30 % poids/poids)). Les clones viraux ont ensuite été classés en différents groupes en effectuant d'autres tests d'hybridation de colonies in situ, en utilisant comme sonde des ADN de plasmides choisis au hasard et contenant un insert ADNc et marqués en$^{32}P$ par nick translation.

IDENTIFICATION DES ADNc. Tirant avantage de la connaissance des attributions de codage des ARNm de NDV (Collins P.L. et al. J. Virol. 43, 1024-1031; 1982), on a effectué la séparation des ARNm par électrophorèse de poly(A)$^+$ -ARNm de cellules infectées en utilisant des gels de 1,5 % d'agarose dans du citrate de sodium 0,025M, pH 3,5, contenant de l'urée 6M et le transfert d'ARN a été effectué sur nitrocellulose. La feuille de nitrocellulose a alors été découpée en plusieurs bandes et chacune d'elles a été hybridée avec un ADNc marqué au $^{32}P$ par nick translation.

L'identification complémentaire des ADNc a été effectuée par la méthode d'hybridation-sélection, telle que décrite par Riezman H. et al. (Embo J. 12; 2161-2168; 1983). Chaque ARNm sélectionné par cette méthode a été traduit dans des oocytes de Xénopus (Gurdon J.B. et al. loc. cit.) et les protéines obtenues ont été immunoprécipitées par des anticorps anti-NDV (Lê Long et al. loc. cit.).

ANALYSE DE LA SEQUENCE ADN. La séquence d'ADN a été déterminée par la méthode chimique de Maxam et Gilbert et par la méthode de séquençage didéoxy de Sanger.

B. RESULTATS

(1) Identification de clones contenant des séquences spécifiques du virus

La Figure 2 montre que, sauf pour un clone (c'est-à-dire le clone I.5 qui s'hybride à un ARNm 35S, qui est un transcript du gène L), chaque ADN s'hybride à une bande importante, un ARNm 18S et à une bande plus faible, localisée dans la région 22S. Comme cette dernière région s'est avérée contenir des transcripts polycistroniques de gènes de NDV (Collins P.L. et al. loc. cit.), les résultats obtenus permettent une identification claire de quatre groupes et, en particulier, ils montrent que le clone II.14 contient au moins une partie de la séquence F (Collins P.L. et al. Loc. cit.).

(2) Séquence nucléotidique du gène de fusion et séquence des acides aminés de la protéine F.

Le clone de ADNc recombinant (II.14) a été choisi pour l'analyse séquentielle d'ADN pour la raison qu'il possède le plus long insert parmi les clones contenant des séquences F spécifiques. Le séquençage a été effectué en suivant la stratégie décrite dans la Figure 3. Plus de 94 % de la séquence de chaque brin de la région codante pour la protéine F ont été déterminés. L'entièreté de la séquence nucléotidique de l'insert du clone II.14, dans le sens ARNm, est indiqué dans la Figure 1. Elle contient 1764 paires de base en excluant la région poly(A) et les queues G-C. Il n'y a qu'une seule longue phase de lecture, partant de l'ATG (nucléotides 68-70) et se terminant avec un codon ocre de terminaison (nucléotides 1646-1648). Ce codon terminal est suivi de quatre terminateurs additionnels en phase.

La région codante code pour une protéine de 526 acides aminés ($M_r$ 56.464, sans tenir compte de la composition en carbohydrate), qui présente trois régions hydrophobes caractéristiques. $F_2$ s'étend probablement du résidu 19 au résidu 98 et est constituée de 80 acides aminés ($M_r$ 8.304 sans tenir compte de la composition en carbohydrate); $F_1$ est un polypeptide de 423 acides aminés (résidus 104-526; $M_r$45.541 sous forme non glycosylée). F présente 4 sites potentiels de glycosylation : un sur $F_2$ et trois sur $F_1$ .

Exemple 2. Expression de la protéine de fusion de NDV dans une levure.

A. MATERIAUX ET METHODES

SOUCHES. 1/ BACTERIENNES. La souche de E. coli utilisée est la souche MM294 (hsdR17, c'est-à-dire $R_K^- m_K^+$). 2/ LEVURE. La souche de S. cerevisiae utilisée est la souche BR10 (MAT alpha, trpl-1, his4-519, his-3, gall,BUP1r) (Butt T.R. et al. Proc. Natl. Acad. Sci. 81; 3332-3336; 1984).

PLASMIDES. Le vecteur navette de levure pCD192 décrit dans la Figure 4 est un plasmide multicopies contenant (i) une partie du plasmide 2-µm bien connu (forme B) comme origine levure de replication, (ii) le gène de levure TRP-1 (Struhl K. et al. Proc. Natl. Acad. Sci. USA 76, 1035-1039; 1979) comme marqueur de sélection dans une levure, (iii) une large fraction du plasmide PBR322 bien connu, comme origine de réplication dans E. coli et un marqueur de sélection (Ap) dans lequel est insérée (iv) la cassette d'expression dérivée de pYSK12 (Butt T.R. et al. loc. cit.) dans laquelle un fragment EcoRI galK a été excisé et remplacé par un groupement de liaison contenant un site de restriction PvuII. (En résumé, la cassette d'expression contient le début du locus CUP-1, c'est-à-dire le promoteur Cu-MT (Butt T.R. et al. loc. cit.) un groupement de liaison EcoRI-PvuII-EcoRI et la terminaison du gène CYCI, c'est-à-dire le terminateur CYCI (Smith M. et al. Cell 16; 753-761; 1979). Ce plasmide contient donc un seul site de restriction PvuII qui est localisé entre le promoteur et le terminateur.

La stratégie pour la construction du plasmide d'expression contenant le gène de fusion NDV est décrite dans la Figure 4. Le mélange de ligation a été utilisé pour transformer la souche E. coli MM294 lui conférant une résistance à l'ampicilline. Les clones ont ensuite été criblés pour détecter la présence de l'insert et pour son orientation vis-à-vis du promoteur, par digestion respectivement par EcoRI et AvaI, permettant l'identification de plasmides présentant l'orientation correcte de F. Un tel plasmide est pYDE1.

TEST DE L'EXPRESSION DU GENE F, SOUS CONTROLE DU PROMOTEUR Cu-MT. La souche de levure BR10 a été transformée par pYDE1 en utilisant des cellules intactes, comme décrit par Ito H. et al. J. Bacteriol. 153; 163-168; 1983; les clones transformés étant sélectionnés sur milieu mimimal sans tryptophane (0,67 % de milieu de base azotée pour levure, 2 % de glucose avec addition d'adénine, d'uracyle et d'histidine (dans chaque cas 20µ/ml). Le promoteur Cu-Mt a alors été induit comme décrit par Butt T.R. et al (loc. cit.) avec cette exception que le temps d'induction était de 2 heures et que l'inducteur était $CuSO_4$ à une concentration finale de 0,1mM. Les extraits cellulaires ont été préparés par traitement avec des billes de verre dans un tampon A (50mM Tris HCl pH 8,0, 2mM EDTA, 0,1mM dithio threitol, 5 % glycerol) (Butt T.R. et al. loc. cit.). Une quantité aliquote de l'extrait total (culot et surnageant) a alors été soumise à une électrophorèse sur un gel de polyacrylamide-SDS à 10 % pour séparer les protéines qui ont ensuite été transférées sur filtre de nitrocellulose. Le transfert s'est effectué pendant 2 heurs sous 250mA dans 0,025M Tris; 0,2M glycine; 20 % méthanol (pH 8,3). Après transfert, le filtre de nitrocellulose a été lavé 3 fois à température ordinaire pendant 5 minutes par trempage dans du tampon PBS (140mM NaCl, 2.5mM KCl, 1,5mM $KH_2PO_4$ , 7,8mM $Na_2HPO_4$ ) et ensuite incubé à 37° C pendant une heure dans du tampon PBS avec 3 % d'albumine sérique bovine. Après 3 lavages de cinq minutes à température ordinaire dans du tampon RIPA (150mM NaCl, 20mM tris-HCl pH 7,5, 10mM EDTA, 0,5 % Triton X-100, 0,1 % SDS), le filtre de nitrocellulose a ensuite été incubé à 37° C pendant 1 heure dans un anticorps polyclonal dirigé contre NDV et dilué 1.000 fois dans du tampon RIPA et ensuite incubé à la même température et pendant la même durée dans 40 µCi de protéine A marquée à $^{125}$ I dans 40 ml de tampon RIPA (la protéine A avait été iodée à une radioactivité spécifique de 35 µCi/µg). Le filtre de nitrocellulose a finalement été lavé six fois avec du tampon RIPA, séché et autoradiographié avec un film Kodak X-AR5.

Il est évident que la référence aux souches et plasmides donnée plus haut est donnée uniquement dans un but d'exemplification étant donné que d'autres souches et plasmides - plasmides navettes pour l'expression dans une levure - peuvent être utilisés dans le même but, comme il bien connu de l'homme de l'art.

B. TEST DE L'EXPRESSION DE F DANS BR10/pYDE1.

Pour déterminer si le polypeptide F-spécifique est produit dans BR10/pYDE1, le promoteur Cu-MT a été induit avec $CuSO_4$ 0,1M pendant 2 heures et des extraits cellulaires ont été préparés et ensuite analysés

dans des essais de transfert (Western blot) avec un anticorps polyclonal dirigé contre NDv (souche Italien). Les résultats sont illustrés dans la Figure 5 qui montre l'apparition de deux bandes d'environ 50 kd, immédiatement en dessous du polypeptide $F_1$ natif alors qu'aucune bande n'est présente dans l'induction du contrôle BR10/pCD192. Il semble donc bien que la protéine F est synthétisée dans la levure sous deux formes de poids moléculaires légèrement différents.

**Revendications**

1. Molécule d'ADN recombinant comprenant une séquence d'ADN codant pour la protéine de fusion du virus de la maladie de Newcastle ou un fragment ou dérivé de celle-ci codant pour un polypeptide qui peut induire une réponse immunitaire à la protéine de fusion du virus de la maladie de Newcastle.

2. Séquence d'ADN de la formule : 5'-

```
        10         20         30         40         50
TCACAACTAT CAGCTGGTGT AACCCCTCGT GCGCTCCAGA TCTTCTACCA
        60         70         80         90        100
GGATCCCGGT ACCTCTAATG CTGATCATCC GAATTGCGCT GACACTGAGC
       110        120        130        140        150
TGTATCCGTC TGACAAGCTC TCTTGATGGC AGGCCTCTTG CAGCTGCAGG
       160        170        180        190        200
GATCGTGGTA ACAGGAGATA AAGCAGTCGA CATATACACC TCATCCCAGA
       210        220        230        240        250
CAGGGTCAAT CATAGTCAAG TTACTCCCAA ATATGCCCAA GGACAAAGAG
       260        270        280        290        300
GCGTGTGCAA AAGCCCCATT GGAGGCATAC AACAGGACAC TGACTACTTT
       310        320        330        340        350
GCTCACCCCC CTTGGCGATT CTATCCGCAG GATACAAGAG TCTGTGACTA
       360        370        380        390        400
CTTCCGGAGG AAGGAGACAG AGACGCTTTA TAGGTGCCAT TATCGGCAGT
       410        420        430        440        450
GTAGCTCTTG GGGTTGCAAC AGCTGCACAG ATAACAGCAG CCTCGGCCCT
       460        470        480        490        500
GATACAAGCC AACCAGAATG CTGCCAACAT CCTCCGGCTT AAGGAGAGCA
       510        520        530        540        550
TTGCTGCAAC CAATGAAGCT GTGCACGAGG TCACTGACGG ATTATCACAA
       560        570        580        590        600
CTAGCAGTGG CAGTAGGGAA GATGCAGCAG TTTGTTAATG ACCAGTTTAA
```

| 610 | 620 | 630 | 640 | 650 |
|---|---|---|---|---|
| TAATACAGCG | CAAGAATTGG | ACTGGTATAA | AAATTGGACA | CAGCAGGTCG |
| 660 | 670 | 680 | 690 | 700 |
| GTGTAGAACT | CAACTTGTAC | CTAACTGAAT | TGACTACAGT | ATTCGGGCCA |
| 710 | 720 | 730 | 740 | 750 |
| CAAATCACTT | CCCCTGCCTT | AACTCCGCTG | ACTATCCAGG | CGCTTTACAA |
| 760 | 770 | 780 | 790 | 800 |
| TTTAGCTGGT | GGTAATATGG | ATTACTTGCT | GACTAAGTTA | GGTGTAGGGA |
| 810 | 820 | 830 | 840 | 850 |
| ACAACCAACT | CAGCTCATTA | ATTGGTAGCG | GCTTGATCAC | CGGCAACCCT |
| 860 | 870 | 880 | 890 | 900 |
| ATTCTGTACG | ACTCACAGAC | TCAGATCTTG | GGTATACAGA | TAACTTCGCC |
| 910 | 920 | 930 | 940 | 950 |
| TTCAGTAGGG | AACCTAAATA | ATATGCGTGC | CACCTACTTG | GAGACCTTAT |
| 960 | 970 | 980 | 990 | 1000 |
| CTGTAAGCAC | AACCAAGGGA | TTTGCCTCAG | CACTTGTCCC | AAAAGTGGTG |
| 1010 | 1020 | 1030 | 1040 | 1050 |
| ACACAGGTCG | GTTCCGTGAT | AGAAGAACTT | GACACCTCAT | ACTGTATGGA |
| 1060 | 1070 | 1080 | 1090 | 1100 |
| GACCGACTTG | GATCTATACT | GTACAAGAAT | AGTGACATTC | CCTATGTCTC |
| 1110 | 1120 | 1130 | 1140 | 1150 |
| CTGGTATTTA | TTCCTGTCTC | AGCGGTAATA | CATCGGCTTG | CATGTATTCA |
| 1160 | 1170 | 1180 | 1190 | 1200 |
| AAGACTGAAG | GCGCACTTAC | TACGCCATAT | ATGGCTCTCA | AAGGCTCAGT |
| 1210 | 1220 | 1230 | 1240 | 1250 |
| TATCGCCAAT | TGCAAGATGA | CAACATGTAG | ATGTGCAGAC | CCCCCGGGTA |
| 1260 | 1270 | 1280 | 1290 | 1300 |
| TCATATCGCA | AAATTATGGA | GAAGCTGTGT | CCTTAATAGA | TAGGCACTCA |
| 1310 | 1320 | 1330 | 1340 | 1350 |
| TGCAATGTCT | TATCCTTAGA | CGGGATAACT | CTGAGACTCA | GTGGGGAATT |
| 1360 | 1370 | 1380 | 1390 | 1400 |
| TGATGCAACC | TATCAAAAGA | ATATCTCAAT | ACTAGATTCT | CAAGTTATAG |
| 1410 | 1420 | 1430 | 1440 | 1450 |
| TGACAGGCAA | TCTCGATATA | TCAACTGAGC | TTGTCAACTC | AATAAGTAAT |

```
      1460       1470       1480       1490       1500
GCCCTGAATA AGTTAGAGGA AAGCAACAGC AAACTAGACA AAGTCAATGT
      1510       1520       1530       1540       1550
CAAACTGACC AGCACATCTG CTCTCATTAC CTACATCGTT TTAACTGTCA
      1560       1570       1580       1590       1600
TATCTCTTGT TTTTGGTGTA CTTAGCCTGG TTCTAGCATG CTACCTGATG
      1610       1620       1630       1640       1650
TACAAGCAAA AGGCACAACA AAAGACCTTA TTATGGCCTT GGGAATAATA
      1660       1670       1680       1690       1700
CCCTTGATCA GATGAGAGCC ACTACAAAAA TATGAATGCA GACGAGAGGC
      1710       1720       1730       1740       1750
GGAGGTATCC CCAATAGCAA TTTGCGTGTA AATTCCGGCA ACCTGTTAAT
      1760
TAAGAAGATT AAAG
```

3′
et fragment ou dérivé de cette séquence qui code pour la protéine de fusion du virus de la maladie de Newcastle ou pour un polypeptide qui peut induire une réponse immunitaire au virus de la maladie de Newcastle.

3. Vecteur permettant une expression dans une levure caractérisé en ce qu'il contient une séquence d'ADN suivant la revendication 1 ou la revendication 2 en aval d'une région de régulation.

4. Le vecteur navette pYDE1.

5. Microorganisme hôte transformé avec au moins une molécule d'ADN recombinant suivant la revendication 1.

6. Protéine de fusion du virus de la maladie de Newcastle ou polypeptide pouvant induire une réponse immunitaire au virus de la maladie de Newcastle caractérisé en ce qu'il est produit par un hôte suivant la revendication 5.

7. Composition stimulant une protection contre une infection par le virus de la maladie de Newcastle caractérisé en ce qu'il comprend au moins une quantité efficace de la protéine de fusion du virus de la maladie de Newcastle ou d'un polypeptide qui peut induire une réponse immunitaire au virus de la maladie de Newcastle et provoque une pénétration cellulaire, caractérisée en ce qu'elle est produite par un hôte suivant la revendication 5.

8. Procédé pour préparer un vecteur suivant la revendication 3, caractérisé en ce qu'on isole le gène F d'un clone d'une banque de ADNc synthétisé au départ de poly(A)$^+$ -ARNm de cellules infectées par NDV et qu'on insère ledit gène F dans un vecteur d'expression en phase et dans l'orientation correcte après un promoteur et avant un terminateur.

9. Procédé suivant la revendication 8 dans laquelle le vecteur d'expression est un vecteur d'expression bactérien.

10. Procédé suivant la revendication 8 dans laquelle le vecteur d'expression est un vecteur pour expression dans une levure.

11. Procédé pour la production de la protéine de fusion du virus de la maladie de Newcastle ou d'un polypeptide qui peut induire une réponse immunitaire au virus de la maladie de Newcastle et provoquer une pénétration cellulaire caractérisé en ce qu'on prépare une molécule d'ADN recombinant suivant la revendication 1 ou 2; on transforme un hôte approprié avec ladite molécule d'ADN recombinant; on cultive l'hôte transformé et on récolte ladite protéine de fusion ou ledit polypeptide.

Revendications pour les Etats Contractants suivants: AT, GR et SP

1. Procédé pour préparer une molécule d'ADN recombinant comprenant une séquence codant pour la protéine de fusion du virus de la maladie de Newcastle ou un fragment ou dérivé de celle-ci dans laquelle le fragment ou dérivé code pour un polypeptide qui peut induire une réponse immunitaire à la protéine de fusion du virus de la maladie de Newcastle caractérisé en ce qu'on isole le gène F d'une clone d'une librairie de ADNc synthétisés au départ de poly(A)$^+$ -ARNm de cellules infectées par NDV.

2. Procédé suivant la revendication 1 caractérisé en ce que la séquence répond à la formule : 5′-

| | | | | |
|---|---|---|---|---|
| 10 | 20 | 30 | 40 | 50 |
| TCACAACTAT | CAGCTGGTGT | AACCCCTCGT | GCGCTCCAGA | TCTTCTACCA |
| 60 | 70 | 80 | 90 | 100 |
| GGATCCCGGT | ACCTCTAATG | CTGATCATCC | GAATTGCGCT | GACACTGAGC |
| 110 | 120 | 130 | 140 | 150 |
| TGTATCCGTC | TGACAAGCTC | TCTTGATGGC | AGGCCTCTTG | CAGCTGCAGG |
| 160 | 170 | 180 | 190 | 200 |
| GATCGTGGTA | ACAGGAGATA | AAGCAGTCGA | CATATACACC | TCATCCCAGA |
| 210 | 220 | 230 | 240 | 250 |
| CAGGGTCAAT | CATAGTCAAG | TTACTCCCAA | ATATGCCCAA | GGACAAAGAG |
| 260 | 270 | 280 | 290 | 300 |
| GCGTGTGCAA | AAGCCCCATT | GGAGGCATAC | AACAGGACAC | TGACTACTTT |
| 310 | 320 | 330 | 340 | 350 |
| GCTCACCCCC | CTTGGCGATT | CTATCCGCAG | GATACAAGAG | TCTGTGACTA |
| 360 | 370 | 380 | 390 | 400 |
| CTTCCGGAGG | AAGGAGACAG | AGACGCTTTA | TAGGTGCCAT | TATCGGCAGT |
| 410 | 420 | 430 | 440 | 450 |
| GTAGCTCTTG | GGGTTGCAAC | AGCTGCACAG | ATAACAGCAG | CCTCGGCCCT |
| 460 | 470 | 480 | 490 | 500 |
| GATACAAGCC | AACCAGAATG | CTGCCAACAT | CCTCCGGCTT | AAGGAGAGCA |

510 520 530 540 550
TTGCTGCAAC CAATGAAGCT GTGCACGAGG TCACTGACGG ATTATCACAA
560 570 580 590 600
CTAGCAGTGG CAGTAGGGAA GATGCAGCAG TTTGTTAATG ACCAGTTTAA
610 620 630 640 650
TAATACAGCG CAAGAATTGG ACTGGTATAA AAATTGGACA CAGCAGGTCG
660 670 680 690 700
GTGTAGAACT CAACTTGTAC CTAACTGAAT TGACTACAGT ATTCGGGCCA
710 720 730 740 750
CAAATCACTT CCCCTGCCTT AACTCCGCTG ACTATCCAGG CGCTTTACAA
760 770 780 790 800
TTTAGCTGGT GGTAATATGG ATTACTTGCT GACTAAGTTA GGTGTAGGGA
810 820 830 840 850
ACAACCAACT CAGCTCATTA ATTGGTAGCG GCTTGATCAC CGGCAACCCT
860 870 880 890 900
ATTCTGTACG ACTCACAGAC TCAGATCTTG GGTATACAGA TAACTTCGCC
910 920 930 940 950
TTCAGTAGGG AACCTAAATA ATATGCGTGC CACCTACTTG GAGACCTTAT
960 970 980 990 1000
CTGTAAGCAC AACCAAGGGA TTTGCCTCAG CACTTGTCCC AAAAGTGGTG
1010 1020 1030 1040 1050
ACACAGGTCG GTTCCGTGAT AGAAGAACTT GACACCTCAT ACTGTATGGA
1060 1070 1080 1090 1100
GACCGACTTG GATCTATACT GTACAAGAAT AGTGACATTC CCTATGTCTC
1110 1120 1130 1140 1150
CTGGTATTTA TTCCTGTCTC AGCGGTAATA CATCGGCTTG CATGTATTCA
1160 1170 1180 1190 1200
AAGACTGAAG GCGCACTTAC TACGCCATAT ATGGCTCTCA AAGGCTCAGT
1210 1220 1230 1240 1250
TATCGCCAAT TGCAAGATGA CAACATGTAG ATGTGCAGAC CCCCCGGGTA
1260 1270 1280 1290 1300
TCATATCGCA AAATTATGGA GAAGCTGTGT CCTTAATAGA TAGGCACTCA
1310 1320 1330 1340 1350
TGCAATGTCT TATCCTTAGA CGGGATAACT CTGAGACTCA GTGGGGAATT
1360 1370 1380 1390 1400

TGATGCAACC TATCAAAAGA ATATCTCAAT ACTAGATTCT CAAGTTATAG

1410 1420 1430 1440 1450

TGACAGGCAA TCTCGATATA TCAACTGAGC TTGTCAACTC AATAAGTAAT

1460 1470 1480 1490 1500

GCCCTGAATA AGTTAGAGGA AAGCAACAGC AAACTAGACA AAGTCAATGT

1510 1520 1530 1540 1550

CAAACTGACC AGCACATCTG CTCTCATTAC CTACATCGTT TTAACTGTCA

1560 1570 1580 1590 1600

TATCTCTTGT TTTTGGTGTA CTTAGCCTGG TTCTAGCATG CTACCTGATG

1610 1620 1630 1640 1650

TACAAGCAAA AGGCACAACA AAAGACCTTA TTATGGCCTT GGGAATAATA

1660 1670 1680 1690 1700

CCCTTGATCA GATGAGAGCC ACTACAAAAA TATGAATGCA GACGAGAGGC

1710 1720 1730 1740 1750

GGAGGTATCC CCAATAGCAA TTTGCGTGTA AATTCCGGCA ACCTGTTAAT

1760

TAAGAAGATT AAAG

3′.ou à un fragment ou dérivé de cette séquence qui code pour la protéine de fusion du virus de la maladie de Newcastle ou pour un polypeptide qui peut induire une réponse immunitaire au virus de la maladie de Newcastle.

3. Procédé pour préparer un vecteur d'expression contenant une séquence suivant la revendication 1 ou 2 en aval d'une région de régulation caractérisé en ce qu'on y insère le gène en phase et dans l'orientation correcte après un promoteur et avant un terminateur.

4. Procédé suivant la revendication 3 dans laquelle le vecteur d'expression est un vecteur d'expression bactérien.

5. Procédé suivant la revendication 3 dans laquelle le vecteur d'expression est un vecteur pour expression dans une levure.

6. Procédé suivant la revendication 5 caractérisé en ce que le vecteur est le vecteur pYDE1;

7. Procédé pour la production de la protéine de fusion du virus de la maladie de Newcastle ou d'un polypeptide qui peut induire une réponse immunitaire au virus de la maladie de Newcastle et provoquer une pénétration cellulaire, caractérisé en ce qu'on prépare une molécule d'ADN recombinant suivant la revendication 1 ou 2; on transforme un hôte approprié avec ladite molécule d'ADN recombinant; on cultive l'hôte transformé et on récolte ladite protéine ou ledit polypeptide.

# Fig.1(a)

TCACAACTATCAGCTGGTGTAACCCCTCGTGCGCTCCAGATCTTCTACCAGGATCCCGGTACCTCTA ATG 70

Met 1

CTG ATC ATC CGA ATT GCG CTG ACA CTG AGC TGT ATC CGT CTG ACA AGC TCT CTT 124

Leu Ile Ile Arg Ile Ala Leu Thr Leu Ser Cys Ile Arg Leu Thr Ser Ser Leu 19

GAT GGC AGG CCT CTT GCA GCT GCA GGG ATC GTG GTA ACA GGA GAT AAA GCA GTC 178

Asp Gly Arg Pro Leu Ala Ala Ala Gly Ile Val Val Thr Gly Asp Lys Ala Val 37

GAC ATA TAC ACC TCA TCC CAG ACA GGG TCA ATC ATA GTC AAG TTA CTC CCA AAT 232

Asp Ile Tyr Thr Ser Ser Gln Thr Gly Ser Ile Ile Val Lys Leu Leu Pro Asn 55

ATG CCC AAG GAC AAA GAG GCG TGT GCA AAA GCC CCA TTG GAG GCA TAC AAC AGG 286

Met Pro Lys Asp Lys Glu Ala Cys Ala Lys Ala Pro Leu Glu Ala Tyr Asn Arg 73

ACA CTG ACT ACT TTG CTC ACC CCC CTT GGC GAT TCT ATC CGC AGG ATA CAA GAG 340

Thr Leu Thr Thr Leu Leu Thr Pro Leu Gly Asp Ser Ile Arg Arg Ile Gln Glu 91

TCT GTG ACT ACT TCC GGA GGA AGG AGA CAG AGA CGC TTT ATA GGT GCC ATT ATC 394

Ser Val Thr Thr Ser Gly Gly Arg Arg Gln Arg Arg Phe Ile Gly Ala Ile Ile 109

GGC AGT GTA GCT CTT GGG GTT GCA ACA GCT GCA CAG ATA ACA GCA GCC TCG GCC 448

Gly Ser Val Ala Leu Gly Val Ala Thr Ala Ala Gln Ile Thr Ala Ala Ser Ala 127

CTG ATA CAA GCC AAC CAG AAT GCT GCC AAC ATC CTC CGG CTT AAG GAG AGC ATT 502

Leu Ile Gln Ala Asn Gln Asn Ala Ala Asn Ile Leu Arg Leu Lys Glu Ser Ile 145

GCT GCA ACC AAT GAA GCT GTG CAC GAG GTC ACT GAC GGA TTA TCA CAA CTA GCA 556

Ala Ala Thr Asn Glu Ala Val His Glu Val Thr Asp Gly Leu Ser Gln Leu Ala 163

GTG GCA GTA GGG AAG ATG CAG CAG TTT GTT AAT GAC CAG TTT AAT AAT ACA GCG 610

Val Ala Val Gly Lys Met Gln Gln Phe Val Asn Asp Gln Phe Asn Asn Thr Ala 181

CAA GAA TTG GAC TGG TAT AAA AAT TGG ACA CAG CAG GTC GGT GTA GAA CTC AAC 664

Gln Glu Leu Asp Trp Tyr Lys Asn Trp Thr Gln Gln Val Gly Val Glu Leu Asn 199

TTG TAC CTA ACT GAA TTG ACT ACA GTA TTC GGG CCA CAA ATC ACT TCC CCT GCC 718

Leu Tyr Leu Thr Glu Leu Thr Thr Val Phe Gly Pro Gln Ile Thr Ser Pro Ala 217

TTA ACT CCG CTG ACT ATC CAG GCG CTT TAC AAT TTA GCT GGT GGT AAT ATG GAT 772

Leu Thr Pro Leu Thr Ile Gln Ala Leu Tyr Asn Leu Ala Gly Gly Asn Met Asp 235

TAC TTG CTG ACT AAG TTA GGT GTA GGG AAC AAC CAA CTC AGC TCA TTA ATT GGT 826

Tyr Leu Leu Thr Lys Leu Gly Val Gly Asn Asn Gln Leu Ser Ser Leu Ile Gly 253

AGC GGC TTG ATC ACC GGC AAC CCT ATT CTG TAC GAC TCA CAG ACT CAG ATC TTG 880

Ser Gly Leu Ile Thr Gly Asn Pro Ile Leu Tyr Asp Ser Gln Thr Gln Ile Leu 271

GGT ATA CAG ATA ACT TCG CCT TCA GTA GGG AAC CTA AAT AAT ATG CGT GCC ACC 934

Gly Ile Gln Ile Thr Ser Pro Ser Val Gly Asn Leu Asn Asn Met Arg Ala Thr 289

TAC TTG GAG ACC TTA TCT GTA AGC ACA ACC AAG GGA TTT GCC TCA GCA CTT GTC 988

Tyr Leu Glu Thr Leu Ser Val Ser Thr Thr Lys Gly Phe Ala Ser Ala Leu Val 307

# Fig.1(b)

```
CCA AAA GTG GTG ACA CAG GTC GGT TCC GTG ATA GAA GAA CTT GAC ACC TCA TAC  1042
Pro Lys Val Val Thr Gln Val Gly Ser Val Ile Glu Glu Leu Asp Thr Ser Tyr   325

TGT ATG GAG ACC GAC TTG GAT CTA TAC TGT ACA AGA ATA GTG ACA TTC CCT ATG  1096
Cys Met Glu Thr Asp Leu Asp Leu Tyr Cys Thr Arg Ile Val Thr Phe Pro Met   343

TCT CCT GGT ATT TAT TCC TGT CTG AGC GGT AAT ACA TCG GCT TGC ATG TAT TCA  1150
Ser Pro Gly Ile Tyr Ser Cys Leu Ser Gly[Asn Thr Ser]Ala Cys Met Tyr Ser   361

AAG ACT GAA GGC GCA CTT ACT ACG CCA TAT ATG GCT CTC AAA GGC TCA GTT ATC  1204
Lys Thr Glu Gly Ala Leu Thr Thr Pro Tyr Met Ala Leu Lys Gly Ser Val Ile   379

GCC AAT TGC AAG ATG ACA ACA TGT AGA TGT GCA GAC CCC CCG GGT ATC ATA TCG  1258
Ala Asn Cys Lys Met Thr Thr Cys Arg Cys Ala Asp Pro Pro Gly Ile Ile Ser   397

CAA AAT TAT GGA GAA GCT GTG TCC TTA ATA GAT AGG CAC TCA TGC AAT GTC TTA  1312
Gln Asn Tyr Gly Glu Ala Val Ser Leu Ile Asp Arg His Ser Cys Asn Val Leu   415

TCC TTA GAC GGG ATA ACT CTG AGA CTC AGT GGG GAA TTT GAT GCA ACC TAT CAA  1366
Ser Leu Asp Gly Ile Thr Leu Arg Leu Ser Gly Glu Phe Asp Ala Thr Tyr Gln   433

AAG AAT ATC TCA ATA CTA GAT TCT CAA GTT ATA GTG ACA GGC AAT CTC GAT ATA  1420
Lys[Asn Ile Ser]Ile Leu Asp Ser Gln Val Ile Val Thr Gly Asn Leu Asp Ile   451

TCA ACT GAG CTT GTC AAC TCA ATA AGT AAT GCC CTG AAT AAG TTA GAG GAA AGC  1474
Ser Thr Glu Leu Val Asn Ser Ile Ser Asn Ala Leu Asn Lys Leu Glu Glu Ser   469

AAC AGC AAA CTA GAC AAA GTC AAT GTC AAA CTG ACC AGC ACA TCT GCT CTC ATT  1528
Asn Ser Lys Leu Asp Lys Val Asn Val Lys Leu_Thr_Ser Thr_Ser_Ala_Leu_Ile   487

ACC TAC ATC GTT TTA ACT GTC ATA TCT [illegible] [illegible] TTT GGT GTA CTT AGC CTG GTT  1582
Thr_Tyr_Ile_Val_Leu_Thr_Val_Ile_Ser_Leu_Val_Phe_Gly_Val_Leu_Ser_Leu_Val   505

CTA GCA TGC TAC CTG ATG TAC AAG CAA AAG GCA CAA CAA AAG ACC TTA TTA TGG  1636
Leu_Ala_Cys_Tyr_Leu_Met_Tyr Lys Gln Lys Ala Gln Gln Lys Thr Leu Leu Trp   523

CCT TGG GAA TAA TACCCTTGATCAGATGAGAGCCACTACAAAAATATGAATGCAGACGAGAGGCGGA  1703
Pro Trp Glu ***                                                           526

GGTATCCCGAATAGCAATTTGCGTGTAAATTCCGGCAACCTGTTAATTAAGAAGATTAAAG             1764
```

Fig. 2

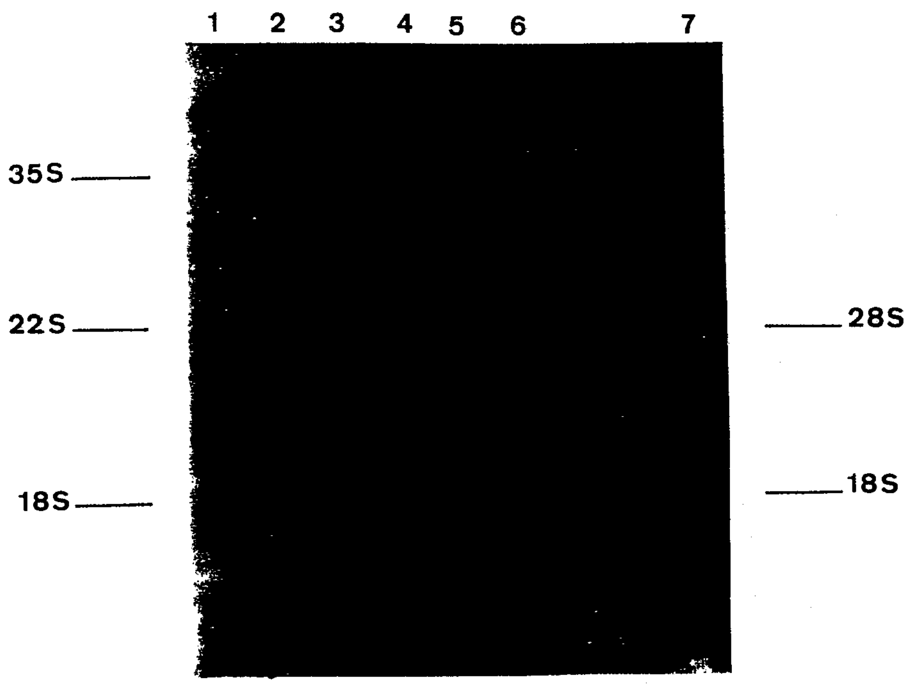

0252060

# Fig.3

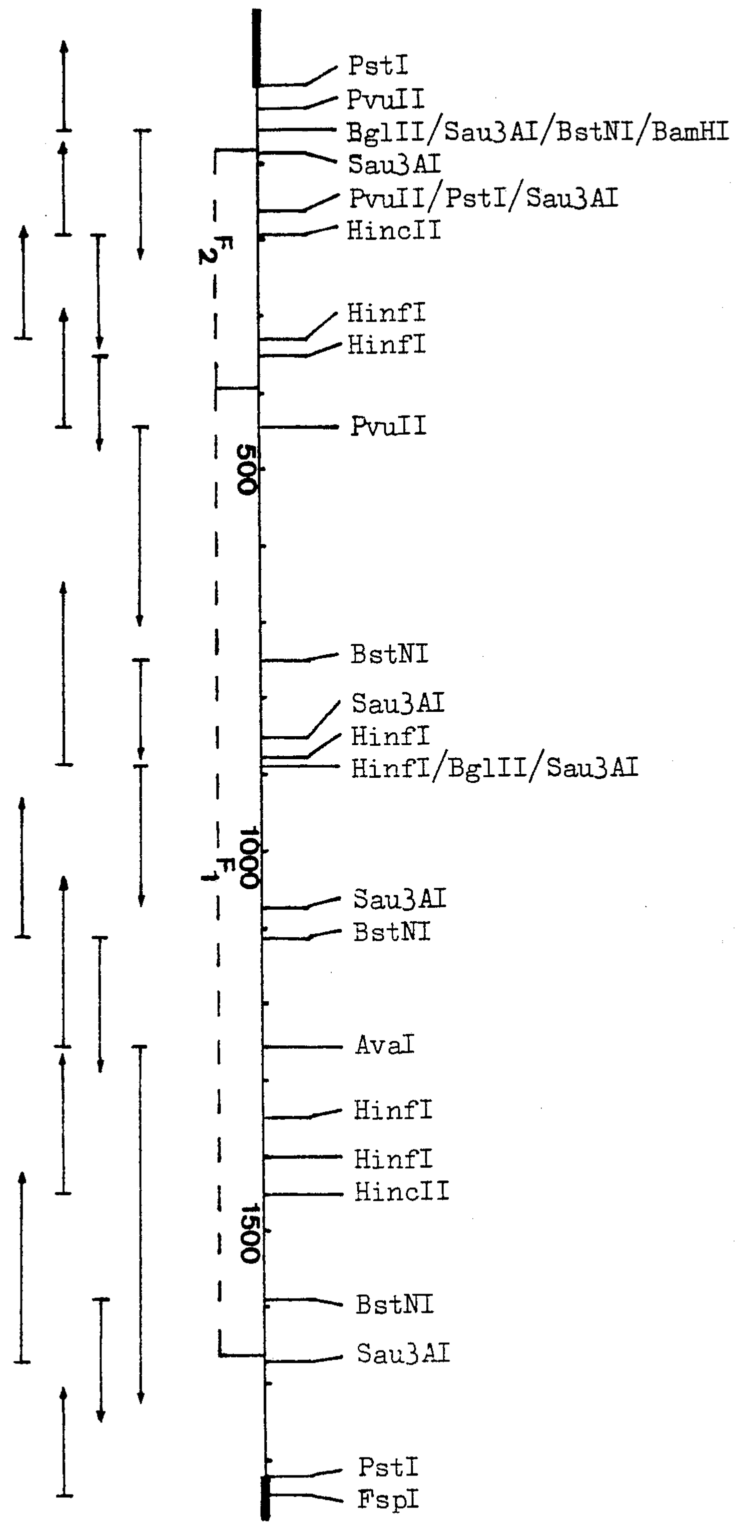

# Fig.4

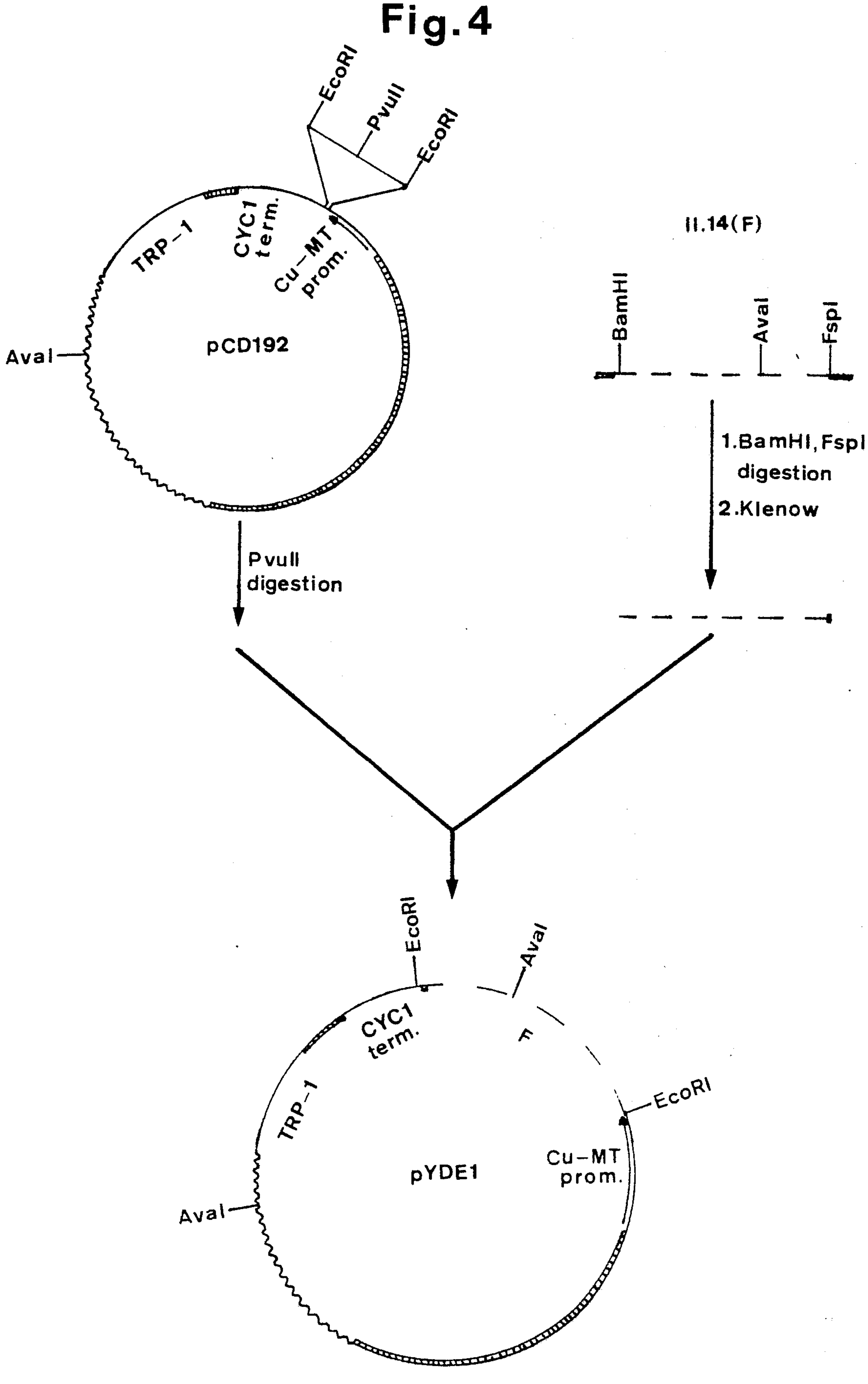
EcoRI
PvuII
EcoRI
TRP-1
CYC1 term.
Cu-MT prom.
AvaI
pCD192
II.14(F)
BamHI
AvaI
FspI
1.BamHI, FspI digestion
2.Klenow
PvuII digestion
EcoRI
AvaI
CYC1 term.
TRP-1
EcoRI
Cu-MT prom.
pYDE1
AvaI

Fig. 5

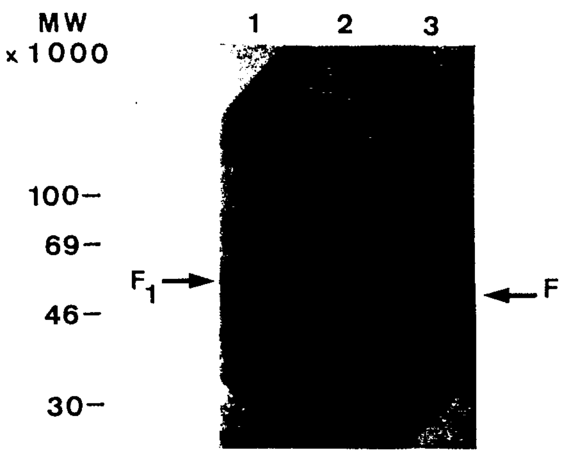

Office européen des brevets

# RAPPORT DE RECHERCHE EUROPEENNE

Numéro de la demande

EP 87 87 0091

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 106, no. 1, 5 janvier 1987, page 113, résumé no. 1125p, Columbus, Ohio, US; L. McGINNES et al.: "Nucleotide sequence of the gene encoding the Newcastle disease virus fusion protein and comparisons of paramyxovirus fusion protein sequences", & VIRUS RES. 1986, 5(4), 343-56 * Abrégé * --- | 1,2 | C 12 N 15/00<br>C 12 P 21/02<br>A 61 K 39/17 |
| X | PROC. NATL. ACAD. SCI. USA, vol. 81, December 1984, page 7683, US; P.L. COLLINS et al.: "Nucleotide sequence of the gene encoding the fusion (F) glycoprotein of human respiratory syncytial virus" * En entier * --- | 1 | **DOMAINES TECHNIQUES RECHERCHES (Int. Cl.4)** |
| P,X | CHEMICAL ABSTRACTS, vol. 106, no. 1, 5 janvier 1987, page 171, résumé no. 97046n, Columbus, Ohio, US; P. CHAMBERS et al.: "Nucleotide sequence of the gene encoding the fusion glycoprotein of Newcastle disease virus", & J. GEN. VIROL. 1986, 67(12), 2685-94 * Abrégé * --- -/- | 1,2 | C 12 N<br>A 61 K |

Le présent rapport de recherche a été établi pour toutes les revendications

| Lieu de la recherche | Date d'achèvement de la recherche | Examinateur |
|---|---|---|
| LA HAYE | 03-09-1987 | SKELLY J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

Office européen des brevets

# RAPPORT DE RECHERCHE EUROPEENNE

Numéro de la demande

EP 87 87 0091



## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| P,X | BIOLOGICAL ABSTRACTS, vol. 84, type 8/4/1, résumé no. 45858, Philadelphia, US; H. SATO et al.: "Molecular cloning and nucleotide sequence of P M and F genes of Newcastle disease virus avirulent strain D26", & VIRUS RES 1987, vol. 7, no. 3, p241-256<br>* Abrégé *<br>--- | 1,2 | |
| E | EP-A-0 227 414 (NATIONAL RESEARCH DEVELOPMENT CORP.)<br>* Page 3, ligne 50 - page 4, ligne 48; pages 11-16; page 17, lignes 21-46; revendications *<br>----- | 1-7 | |
| | | | DOMAINES TECHNIQUES RECHERCHES (Int. Cl.4) |

Le présent rapport de recherche a été établi pour toutes les revendications

| Lieu de la recherche | Date d'achèvement de la recherche | Examinateur |
|---|---|---|
| LA HAYE | 03-09-1987 | SKELLY J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82